(19) Europäisches Patentamt
European Patent Office
Office européen des brevets

(11) EP 2 742 971 B1

(12) FASCICULE DE BREVET EUROPEEN

(45) Date de publication et mention
de la délivrance du brevet:
**21.10.2015 Bulletin 2015/43**

(51) Int Cl.:
*A61N 1/368* (2006.01)       *A61B 5/00* (2006.01)
*A61B 5/04* (2006.01)        *A61B 5/0464* (2006.01)
*A61N 1/37* (2006.01)         *G06K 9/00* (2006.01)

(21) Numéro de dépôt: **13185172.7**

(22) Date de dépôt: **19.09.2013**

(54) **Dispositif médical implantable actif de type stimulateur cardiaque avec test de capture par analyse simplifiée d'un vectogramme**

Aktive implantierbare medizinische Vorrichtung vom Typ Herzschrittmacher mit Nachweistest durch vereinfachte Analyse eines Vektogramms

Active implantable medical device such as a pacemaker with capture testing by simplified analysis of a vector cardiogram

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.12.2012 FR 1262073**

(43) Date de publication de la demande:
**18.06.2014 Bulletin 2014/25**

(73) Titulaire: **Sorin CRM SAS**
**92140 Clamart Cedex (FR)**

(72) Inventeurs:
• **Euzen, Marie-Anen**
**91570 Bievres (FR)**
• **Vincent, Elodie**
**92160 Antony (FR)**

(74) Mandataire: **Dupuis-Latour, Dominique**
**Bardehle Pagenberg**
**10, boulevard Haussmann**
**75009 Paris (FR)**

(56) Documents cités:
**EP-A1- 2 324 885**       **EP-A2- 1 995 685**
**US-A1- 2006 253 164**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

L'invention concerne les "dispositifs médicaux implantables actifs" tels que définis par la Directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

La stimulation antibradycardique implique la délivrance contrôlée d'impulsions à l'oreillette et/ou au ventricule (dispositifs de type simple ou double chambre). Dans le cas des thérapies de resynchronisation cardiaque CRT, la stimulation doit en outre être appliquée conjointement aux deux ventricules (dispositifs de type multisite).

De façon générale, après la stimulation d'une cavité il est important de recueillir l'"onde évoquée", c'est-à-dire l'onde de dépolarisation induite par la stimulation de cette cavité, afin de déterminer si la stimulation a été efficace ou non. Ce test ("test de capture") est notamment utilisé pour ajuster l'amplitude et/ou la largeur des impulsions de stimulation, c'est-à-dire l'énergie délivrée au site de stimulation.

Il existe de nombreuses techniques pour effectuer ce test de capture, par exemple celle décrite dans le WO 93/02741 A1 ou US 5 411 533 A (ELA Medical). Dans le dispositif décrit par ce document, le test du seuil d'efficacité de la stimulation ou "seuil d'entrainement" est effectué à intervalles réguliers, par exemple toutes les six heures, par mise en oeuvre d'un algorithme de test automatique. L'amplitude des impulsions de stimulation est ensuite ajustée sur la base du seuil ainsi mesuré, avec une marge de sécurité additionnelle pour tenir compte notamment des aléas sur la détermination du seuil.

Le EP 1 287 849 A1 (ELA Medical) décrit une technique d'ajustement "cycle à cycle", consistant à opérer le test de capture, et le réajustement éventuel de l'énergie de stimulation, non plus à intervalles réguliers (par exemple toutes les six heures) mais en continu en vérifiant à chaque cycle si la stimulation a été efficace ou non, et de ce fait de manière beaucoup plus réactive.

Le EP 2 324 885 A1 (Sorin CRM) procède par analyse de signaux d'électrogramme endocavitaire (signaux EGM) recueillis concurremment sur deux voies distinctes en provenance d'une même cavité, plus particulièrement du ventricule, pour dériver des paramètres pertinents relatifs à l'origine du signal recueilli, notamment l'onde de dépolarisation cardiaque pour détecter la présence ou l'absence d'une capture.

Les deux voies EGM différentes peuvent en particulier être celle d'un signal unipolaire (signal lointain ou *far-field* recueilli entre le boitier et une électrode distale ou bien proximale de la sonde), et celle d'un signal bipolaire (signal proche ou *near-field* recueilli entre une électrode distale et une électrode proximale de cette même sonde).

L'analyse de ces signaux est une analyse bidimensionnelle à partir de la "boucle cardiaque" ou "vectogramme" (VGM), qui est la représentation dans un espace à deux dimensions de l'un de ces deux signaux par rapport à l'autre. Cet espace est typiquement un espace "voie unipolaire (en ordonnée) vs. voie bipolaire (en abscisse)", chaque battement ou fraction significative de battement étant alors représenté par son vectogramme dans le plan ainsi défini - et en faisant donc abstraction de la dimension temporelle.

On soulignera que ce "vectogramme" (VGM), qui est obtenu à partir de signaux d'électrogramme (EGM) issus de sondes intracardiaques, ne doit pas être confondu avec le "vectocardiogramme" (VCG) qui est, lui, obtenu à partir de signaux d'électrocardiogramme (ECG) issus d'électrodes externes placées sur le thorax du patient.

L'analyse du vectogramme pour le test de capture peut être une analyse intrinsèque des propriétés de la boucle cardiaque, considérée en tant que telle sur le cycle à analyser : pour ce faire, l'algorithme calcule et analyse des paramètres descripteurs du vectogramme, qui peuvent être notamment les angles des vecteurs tangents respectifs considérés en divers points de la caractéristique 2D, ou encore la courbure de cette caractéristique 2D, ou bien une combinaison de plusieurs paramètres, par exemple une combinaison de la norme et de l'angle des vecteurs tangents.

Mais de préférence l'analyse du vectogramme est une analyse comparative, consistant à rechercher une corrélation entre d'une part les caractéristiques du vectogramme du cycle à analyser, et d'autre part les mêmes caractéristiques relevées sur un ou plusieurs cycles de référence obtenus dans des conditions parfaitement déterminées : capture, absence de capture, fusion... Par exemple les vecteurs tangents obtenus pour un cycle cardiaque à analyser sont comparés aux mêmes vecteurs observés pour des courbes de référence, obtenues au préalable, sur une durée identique dans des situations respectivement de capture ou d'absence de capture ; l'algorithme de caractérisation évalue un coefficient de corrélation entre les paramètres descripteurs du cycle à analyser et des cycles de référence, puis discrimine entre capture et perte de capture en fonction des résultats du calcul de corrélation, éventuellement combinés à d'autres critères de décision, notamment l'angle moyen entre les vecteurs tangents respectifs du vectogramme analysé et du vectogramme de référence. Cette technique se révèle efficace en particulier à l'égard des cycles atypiques comme dans les situations de fusion, lorsqu'une stimulation est déclenchée de façon concomitante à une dépolarisation spontanée pendant le test de capture.

Elle n'est toutefois pas dépourvue d'inconvénients.

Un *premier inconvénient* est le niveau des ressources logicielles nécessaires pour la mise en oeuvre de l'algorithme de caractérisation du vectogramme, qui nécessite des moyens de calcul relativement importants, et qui doit être mis en oeuvre en temps réel dans le cas d'un test de capture cycle-à-cycle. Les besoins en calcul sont difficilement compatibles avec ce dont il est possible de disposer dans un implant conventionnel, dont le processeur et les mémoires sont sollicités par la mise en oeuvre de très nombreuses fonctions de détection et de calcul.

Le besoin subsiste donc de disposer d'une méthode simple, mais efficace, de caractérisation d'un vectogramme pour un test de capture.

Un *second inconvénient* est la nécessité de disposer de plusieurs vectogrammes de référence qui feront l'objet du calcul de corrélation avec le vectogramme courant à analyser. Ces vectogrammes de référence sont obtenus soit manuellement, par un test déclenché par le praticien qui valide ensuite chaque type de référence (capture complète sur tous les sites stimulés, capture partielle de certains sites seulement, perte de capture totale, etc.) soit de façon automatique pour les vectogrammes correspondant à une capture complète et à des pertes partielles ou complètes de capture. Dans le cas où les vectogrammes de référence sont établis de façon automatique, le dispositif effectue régulièrement (par exemple toutes les 4 heures, toutes les semaines...) des tests de stimulation à énergie élevée ou à zéro volt sur les différents sites, de manière à mettre à jour les vectogrammes de référence.

Il subsiste ainsi également le besoin de disposer d'une méthode simple de test de capture qui puisse faire abstraction des vectogrammes de référence, en se passant donc de la technique relativement complexe d'obtention et de mise à jour de ces derniers.

L'idée de base de l'invention consiste à examiner le vectogramme par rapport à un domaine prédéterminé défini dans le repère où il est tracé, par exemple par rapport à un domaine rectangulaire de dimensions et de position prédéfinies, et de procéder à une analyse topologique de la distribution des points du vectogramme par rapport à ce domaine : la position de ces points à l'intérieur ou à l'extérieur du domaine prédéterminé constituera le critère utilisé pour décider de la présence ou de l'absence d'une capture.

Plus précisément, l'invention propose un dispositif comprenant des moyens de test de capture du type général décrit par le EP 2 324 885 A1 précité, c'est-à-dire un dispositif comprenant : des moyens de délivrance d'impulsions électriques de stimulation appliquées à des électrodes aptes à être implantées dans au moins une cavité cardiaque d'un patient ; des moyens de recueil de l'activité électrique du coeur, comprenant des moyens pour produire au moins deux composantes temporelles distinctes à partir de deux signaux EGM distincts d'électrogramme endocavitaire recueillis concurremment ; et des moyens de test de capture, pour détecter la survenue d'une onde de dépolarisation induite par la stimulation de la cavité sur au moins un cycle stimulé. Les moyens de test de capture comprennent : des moyens pour déterminer une caractéristique 2D non-temporelle représentative du cycle cardiaque à analyser, à partir des variations de l'une des composantes temporelles en fonction de l'autre ; et des moyens discriminateurs, pour déterminer la présence ou l'absence d'une capture par analyse de la caractéristique 2D non-temporelle.

De façon caractéristique de l'invention, les moyens discriminateurs comprennent : des moyens d'analyse topologique, pour déterminer si la caractéristique 2D non-temporelle est ou non incluse dans un domaine prédéterminé défini dans un repère correspondant à l'espace des deux composantes temporelles, et décider i) l'absence de capture dans le premier cas et ii) la présence d'une capture dans le second cas.

Selon diverses caractéristiques subsidiaires avantageuses :

- le domaine est un domaine rectangulaire, et il est centré sur le point d'origine du repère correspondant à l'espace des deux composantes temporelles ;
- la caractéristique 2D non-temporelle est une caractéristique 2D échantillonnée décrite par une série de points discrets successifs, et les moyens d'analyse topologique sont aptes à analyser la position relative de chaque point par rapport au domaine ;
- les moyens d'analyse topologique sont aptes à décider que la caractéristique 2D n'est pas incluse dans le domaine dès lors qu'au moins un point de la caractéristique 2D échantillonnée se situe hors du domaine, ou bien dès lors qu'au moins deux points de la caractéristique 2D échantillonnée se situent hors du domaine, notamment au moins deux points consécutifs ;
- la caractéristique 2D non-temporelle est déterminée pour une pluralité de cycles cardiaques successifs, et les moyens d'analyse topologique sont aptes à décider l'absence de capture si au moins l'une des caractéristiques ainsi déterminées est incluse dans le domaine, et la présence d'une capture dans le cas contraire ;
- les moyens pour déterminer la caractéristique 2D non-temporelle sont des moyens aptes à déterminer cette caractéristique à partir des variations des composantes temporelles sur une fraction du cycle cardiaque à analyser, dans une fenêtre temporelle d'analyse ouverte à l'instant de la stimulation ou décalée par rapport à cet instant, de manière à isoler le complexe QRS du battement cardiaque des pics de stimulation ventriculaire et/ou auriculaire qui le précèdent ;
- pour un test de capture consistant à détecter la survenue d'une onde de dépolarisation induite dans une cavité par la stimulation de cette même cavité, le dispositif comprend en outre : des moyens pour raccourcir temporairement le délai atrioventriculaire pendant l'activation des moyens de test de capture, de sorte qu'une contraction ventriculaire spontanée survenant après une absence de capture se situe hors de la fenêtre temporelle d'analyse ;

- pour un test de capture consistant à détecter la survenue d'une onde de dépolarisation induite dans une cavité par la stimulation d'une autre cavité, le dispositif comprend en outre : des moyens pour raccourcir temporairement le délai atrioventriculaire pendant l'activation des moyens de test de capture ; et des moyens pour conditionner l'activation des moyens de test de capture au respect du critère : AR (ou PR) $\geq$ DAV + VV + M, AR (ou PR) étant l'intervalle séparant la stimulation (ou la détection) auriculaire de la dépolarisation ventriculaire subséquente, DAV étant la durée du délai atrioventriculaire, VV étant le délai de conduction entre les deux cavités, c'est-à-dire la durée que met la dépolarisation issue de la stimulation d'une cavité à se propager et être détectable dans la cavité non stimulée, et M étant une constante correspondant à la différence minimale admissible entre DAV+VV et AR (ou PR), de sorte qu'une contraction ventriculaire spontanée survenant après une absence de capture se situe hors de la fenêtre temporelle d'analyse ;

- les signaux EGM recueillis concurremment sur des voies respectives distinctes comprennent un signal *near-field* de composante bipolaire recueilli entre une électrode proximale et une électrode distale d'une sonde apte à être placée dans une cavité cardiaque, et un signal *far-field* de composante unipolaire recueilli entre le boîtier du dispositif et l'électrode proximale ou distale de la sonde.

On va maintenant décrire un exemple de mise en oeuvre de l'invention, en référence aux dessins annexés où les mêmes références numériques désignent d'une figure à l'autre des éléments identiques ou fonctionnellement semblables.

La Figure 1 est une vue générale montrant notamment une sonde bipolaire implantée au fond du ventricule droit du coeur.

La Figure 2 illustre les signes EGM obtenus respectivement sur les voies bipolaire ventriculaire, et unipolaire ventriculaire, de la sonde de la Figure 1.

La Figure 3 est un exemple de signaux EGM obtenus dans une configuration de stimulation biventriculaire, montrant la prédominance du signal bipolaire par rapport au signal unipolaire.

La Figure 4 est un autre exemple de signaux EGM obtenus dans une configuration de stimulation du seul ventricule droit, montrant la prédominance du signal unipolaire par rapport au signal bipolaire.

La Figure 5 illustre la manière de combiner entre eux les signaux bipolaire et unipolaire de la Figure 2 pour obtenir une caractéristique 2D de type vectogramme (VGM).

La Figure 6 est un exemple de vectogramme obtenu à partir d'EGMs issus de la même cavité que celle où est appliquée la stimulation.

La Figure 7 est un exemple de vectogramme obtenu à partir d'EGMs issus d'une autre cavité que celle où est appliquée la stimulation.

Les Figures 8a et 8b illustrent les vectogrammes obtenus, respectivement en présence et en l'absence d'une capture, et la manière selon l'invention de discriminer entre ces deux situations par analyse topologique du vectogramme.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* et *Paradym* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, au moyen d'algorithmes appropriés exécutés par un micro-contrôleur ou un processeur numérique de signal.

Comme cela a été indiqué plus haut, la technique d'analyse de l'invention consiste à détecter l'onde évoquée consécutive à la stimulation d'une cavité à partir de signaux d'électrogramme (EGM) recueillis sur deux voies distinctes et analysés en deux dimensions.

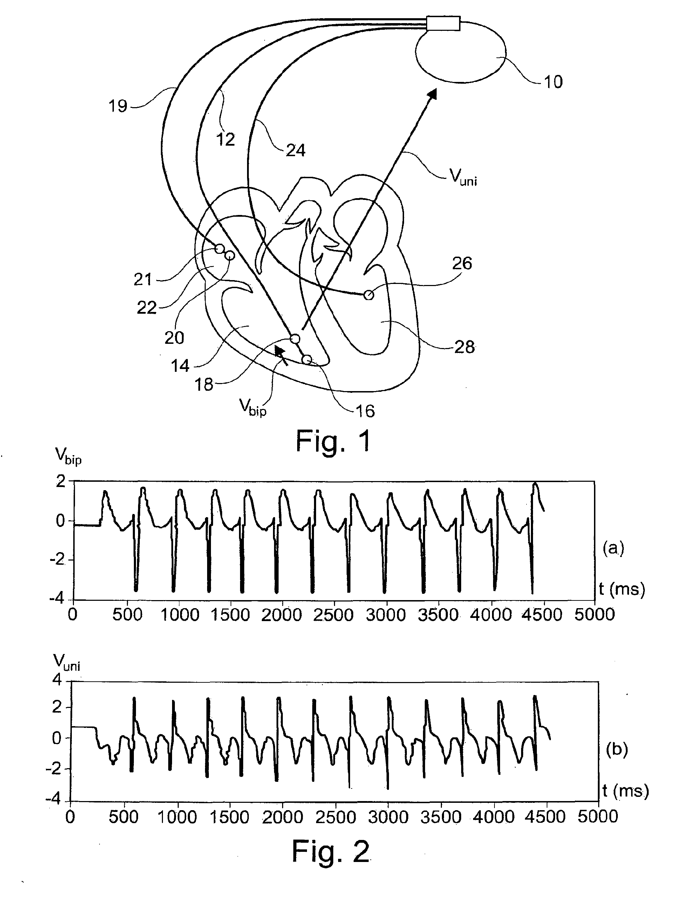

La Figure 1 illustre une configuration de stimulation dans laquelle un générateur d'impulsions 10 est associé à une sonde 12 implantée dans le ventricule droit 14. La tête de sonde comporte deux électrodes, à savoir une électrode distale 16 et une électrode proximale 18, ce qui permet de recueillir un premier électrogramme Vbip correspondant à la différence de potentiel mesurée entre l'électrode distale 16 et l'électrode proximale 18, et d'autre part un second électrogramme *Vuni,* mesuré par la différence de potentiel entre l'une des électrodes, par exemple l'électrode proximale 18, et le boitier métallique du générateur 10.

Une sonde auriculaire 19, pourvue d'électrodes de détection distale et proximale 20, 21 est placée dans l'oreillette droite 22 pour la détection des signaux dans cette cavité et l'application éventuelle d'une stimulation auriculaire.

Dans le cas d'une stimulation biventriculaire, destinée notamment à rétablir la synchronisation entre les deux ventricules, le dispositif est pourvu d'une deuxième sonde ventriculaire 24, par exemple une sonde disposée dans le réseau coronaire et comportant une électrode 26 disposée au voisinage du ventricule gauche 28. Cette électrode ventriculaire gauche 26 permet notamment d'assurer la stimulation concomitante des deux ventricules droit et gauche pour rétablir la synchronisation entre ces deux cavités et améliorer l'hémodynamique générale du patient.

On notera que, dans le cas d'un défibrillateur, le signal bipolaire *Vbip* peut être également recueilli entre l'une des électrodes 16 et 18 et le bobinage ou *coil* ventriculaire formant électrode de défibrillation, le signal unipolaire *Vuni* étant alors recueilli entre le boitier métallique 10 et ce coil ventriculaire.

La Figure 2 illustre un exemple de tracés d'électrogramme *Vbip* et *Vuni* observés respectivement sur la voie bipolaire ventriculaire (Figure 2a) et sur la voie unipolaire ventriculaire (Figure 2b) de la configuration de la Figure 1.

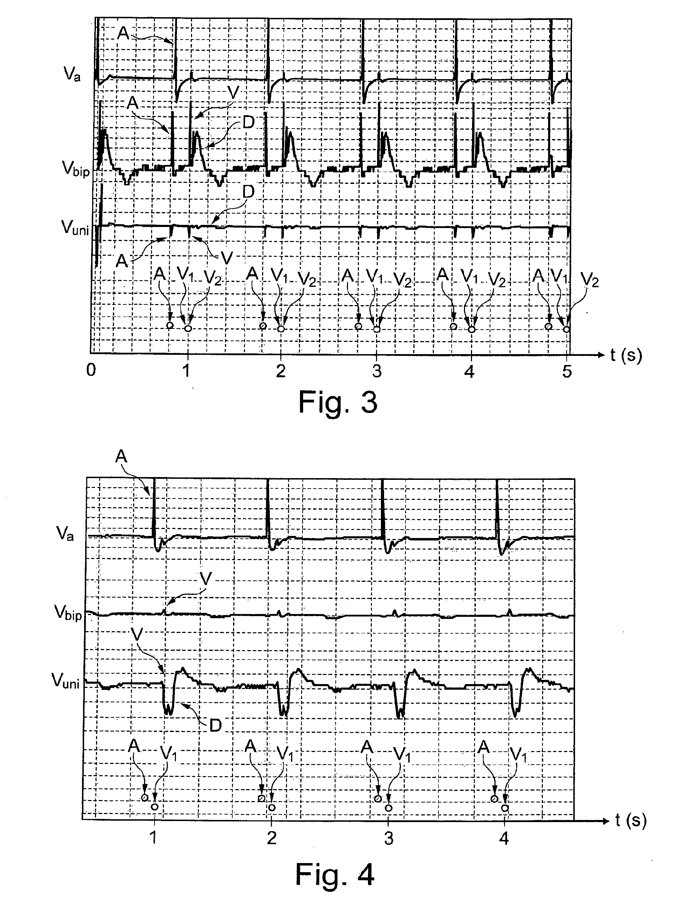

La Figure 3 est un exemple de signaux EGM obtenus dans une configuration de stimulation biventriculaire, montrant la prédominance du signal bipolaire *Vbip* par rapport au signal unipolaire *Vuni.*

On a représenté sur cette figure les chronogrammes des signaux EGM ventriculaires *Vbip* et *Vuni* ainsi que le signal EGM auriculaire *Va.* De plus, des marqueurs A et V1, V2 indiquent les instants d'application des stimulations auriculaire et des stimulations ventriculaires (dans cet exemple, concomitantes pour les deux ventricules droit et gauche).

Cinq cycles sont visibles, correspondant tous à des stimulations capturantes. En examinant cette figure, on notera que le signal EGM *far-field* de composante unipolaire *Vuni* comprend un pic de stimulation auriculaire A, un pic de stimulation ventriculaire V, et une dépolarisation D. Cette dépolarisation D est toutefois pratiquement inexistante sur le signal *Vuni,* de sorte qu'un algorithme de discrimination basé uniquement sur la dépolarisation au niveau de ce signal considèrerait - à tort - qu'il y a eu perte de capture. En revanche, le signal EGM *near-field* de composante bipolaire *Vbip* présente une résolution correcte (amplitude de l'ordre de 2 V de la dépolarisation D), et serait suffisant pour déterminer la présence effective d'une capture.

La Figure 4 est un autre exemple de signaux EGM obtenus dans une configuration de stimulation du seul ventricule droit, montrant la prédominance du signal unipolaire *Vuni* par rapport au signal bipolaire *Vbip.*

Les quatre cycles visibles sur ces chronogrammes correspondent à des stimulations toutes capturantes. En revanche, la situation est opposée à celle de l'exemple de la Figure 3 : on peut en effet observer que la dépolarisation ventriculaire sur le signal *Vbip* est d'amplitude et de largeur très faibles (moins de 1 V), ce qui pourrait induire en erreur un algorithme de discrimination de la capture basée sur l'analyse de ce signal. En revanche, le signal *Vuni* présente une qualité et une amplitude suffisantes au niveau de la dépolarisation D pour être correctement interprété par l'algorithme, qui avèrerait la présence d'une capture.

Ainsi, l'utilisation d'une seule voie EGM, *Vbip* ou bien *Vuni,* pour vérifier la capture sur un cycle stimulé peut entrainer des erreurs dans l'appréciation de la présence ou la perte de capture, en particulier lorsque l'EGM est de mauvaise qualité.

Cependant, on constate généralement que même si une voie EGM est de piètre qualité, une autre voie EGM, provenant de la même cavité, peut se révéler de qualité suffisante.

L'invention prévoit donc, pour pallier cet inconvénient, de recueillir au moins deux composantes temporelles distinctes (en l'espèce, les signaux EGM *Vbip* et *Vuni*) provenant de la même cavité, généralement le ventricule droit.

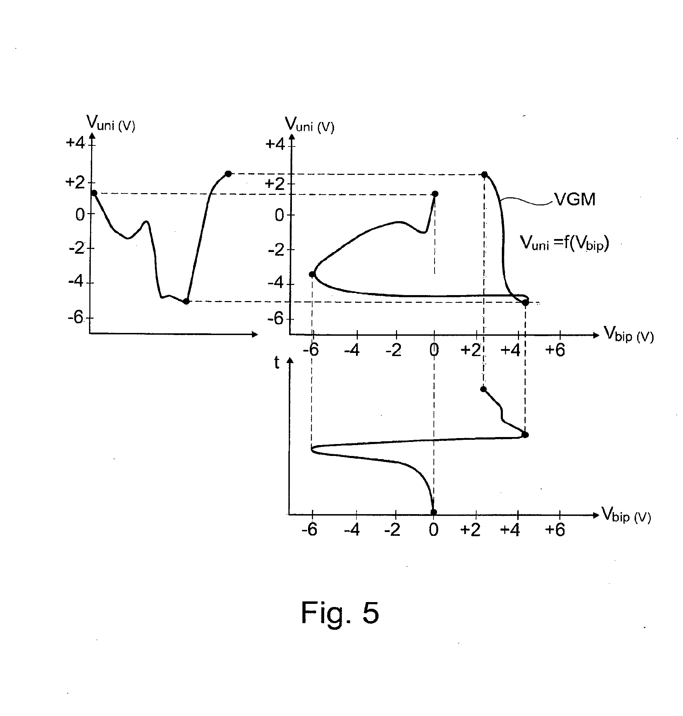

Ces deux composantes temporelles *Vbip* et *Vuni* ont été illustrées sur la Figure 5, qui montre la manière de les combiner entre elles pour obtenir une caractéristique 2D de type vectogramme (VGM).

Concrètement, les signaux EGM $Vuni(t)$ et $Vbip(t)$ recueillis sont échantillonnés et les échantillons successifs des deux composantes ainsi recueillis sont mémorisés puis combinés entre eux pour éliminer la variable temporelle $t$ et produire une courbe paramétrique (vectogramme VGM) du type $Vuni = f(Vbip)$.

Cette courbe $Vuni = f(Vbip)$ est une courbe paramétrique sans dimension temporelle, tracée à partir des variations de l'une des composantes temporelles (*Vuni*) en fonction de l'autre (*Vbip*).

Elle constitue une caractéristique de type vectocardiogramme (VGM) représentative du cycle cardiaque à analyser (ou d'une fraction de ce cycle), et sera désignée par la suite "caractéristique 2D non-temporelle". Elle présente graphiquement la forme d'une boucle, et le temps n'apparait plus que dans la manière dont la boucle est parcourue sur la durée du cycle ou de la fraction de cycle.

On notera incidemment que l'analyse 'bidimensionnelle" ou "en deux dimensions" (2D) évoquée ici ne doit pas être entendue de manière en elle-même limitative. L'invention peut en effet s'appliquer aussi bien à une analyse dans un espace multidimensionnel d'ordre supérieur (3D ou plus), par extrapolation des enseignements de la présente description à une situation où des signaux EGM provenant d'une même cavité sont recueillis simultanément sur trois voies ou plus.

Par ailleurs, il n'est pas indispensable d'analyser la totalité du cycle, l'analyse d'une fraction significative de ce cycle (typiquement, celle débutant au moment de la stimulation, ou bien centrée autour du complexe QRS) étant en général suffisante pour permettre la détection de l'onde évoquée et discriminer entre cycle capturant et cycle non capturant.

Dans ce cas, la caractéristique curviligne représentative du VGM n'est pas une boucle fermée, dans la mesure où elle ne correspond qu'à une partie de la boucle cardiaque complète, à savoir le complexe QRS isolé à l'intérieur d'une fenêtre d'analyse donnée.

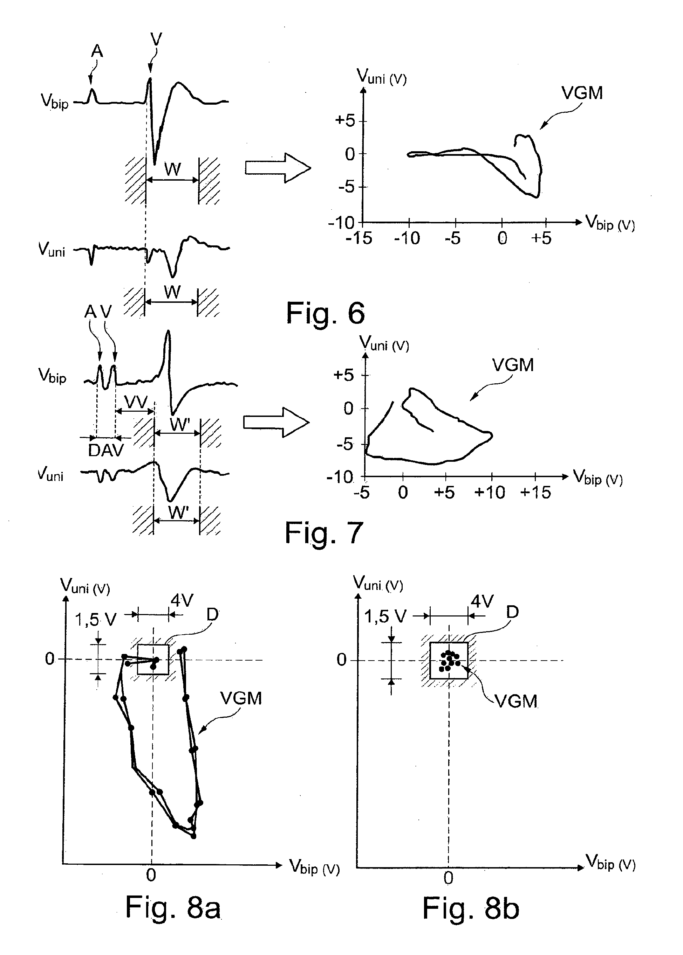

Les Figures 6 et 7 sont des exemples de vectogrammes obtenus à partir d'EGM issus respectivement de la même cavité que celle où est appliquée la stimulation, ou d'une autre cavité que celle où est appliquée la stimulation.

En effet, un test de capture ventriculaire est une analyse de cycles ventriculaires stimulés, mais il est possible de considérer soit les EGM *Vuni* et *Vbip* de la cavité stimulée, soit les EGMs *Vuni* et *Vbip* provenant d'une autre cavité.

La Figure 6 illustre le cas où les EGMs proviennent de la même cavité que la stimulation. : dans ce cas, les échantillons utilisés pour tracer le VGM sont pris dans une fenêtre VV, par exemple de 63 ms à partir de l'instant V de stimulation ventriculaire (le début de cette fenêtre pouvant être éventuellement décalé, jusqu'à 30 ms, si l'on veut éviter que le pic de stimulation V ne soit présent dans la fenêtre et donc sur la caractéristique VGM à analyser).

La Figure 7 correspond à l'autre cas, où les EGM *Vbip* et *Vuni* proviennent d'une autre cavité que celle où la stimulation a lieu : les points utilisés sont alors pris dans une fenêtre VV', par exemple de 63 ms centrée sur l'onde de dépolarisation. La fenêtre est centrée sur l'onde de dépolarisation correspondant à un cycle stimulé qui présente une capture avérée. Cette fenêtre reste la même pour toute la suite du test de capture ; en cas de perte de capture, la fenêtre ne contiendra plus de dépolarisation, car la dépolarisation spontanée arrivera plus tard, après la fin de la fenêtre.

Lorsque la stimulation est opérée de façon concomitante dans deux cavités, typiquement dans le cas d'une stimulation biventriculaire, le délai entre les deux stimulations ventriculaires est généralement faible, ou nul, de sorte que la stimulation d'une cavité ne sera pas conduite dans la seconde cavité. Il convient alors de se baser sur les EGM *Vbip* et *Vuni* d'une première cavité pour déterminer si la stimulation dans cette cavité a été ou non capturante, et de même pour la seconde cavité.

En revanche, si le délai entre les stimulations des deux cavités est important et que la stimulation d'une première cavité est conduite dans l'autre cavité avant que la stimulation de cette dernière cavité n'ait lieu, la situation est comparable à celle que l'on aurait avec une stimulation unique dans la première cavité.

En tout état de cause, il est nécessaire que le dispositif se trouve dans une condition telle que, en cas de perte de capture mais avec un cycle ventriculaire spontané présent malgré l'absence de capture, ce cycle spontané n'apparaisse pas dans la fenêtre utilisée pour tracer la caractéristique VGM.

Pour ce faire, il convient de programmer le dispositif avec un délai auriculo-ventriculaire DAV très court pendant la durée du test de capture.

Cependant, cette modification peut s'avérer insuffisante dans le cas notamment où les signaux EGM proviennent d'une autre cavité que celle où la stimulation a eu lieu : il existe donc un délai entre l'instant de la stimulation dans une première cavité (l'un des ventricules) et celui de la dépolarisation observée sur les signaux EGM *Vbip* et *Vuni* de la seconde cavité (l'autre ventricule).

Comme on l'a indiqué plus haut, la fenêtre utilisée pour tracer la représentation VGM est une fenêtre centrée sur la dépolarisation correspondant à une stimulation capturante.

Pour s'assurer qu'aucune dépolarisation spontanée ne soit présente dans cette fenêtre, outre le raccourcissement temporaire du DAV programmé, il est nécessaire pour démarrer le test de capture que la condition suivante soit vérifiée (en partant de l'hypothèse que les deux cavités sont des cavités ventriculaires) :

$$AR \ (ou \ PR) \geq DAV + VV + M,$$

AR (ou PR) étant l'intervalle séparant la stimulation (ou la détection) auriculaire de la dépolarisation ventriculaire subséquente,
DAV étant la durée du délai atrioventriculaire,
VV étant le délai de conduction entre les deux cavités, c'est-à-dire la durée que met la dépolarisation issue de la stimulation d'une cavité à se propager et être détectable dans la cavité non stimulée, et M étant une constante correspondant à la différence minimale admissible entre DAV+VV et AR (ou PR), par exemple M = 63 ms.

La fenêtre centrée sur la dépolarisation est une fenêtre qui commence à un instant situé à (DAV + VV) par rapport à la dépolarisation auriculaire A (ou P), et qui se termine à (DAV + VV + 63 ms), comme illustré Figure 7. Dès lors que ces diverses conditions sont vérifiées pendant la durée du test de capture, on est certain que la présence d'une dépolarisation révèlera une capture effective, tandis que l'absence de dépolarisation indiquera une perte de capture.

Le point de départ de l'invention réside dans la constatation qu'en cas de capture la caractéristique VGM, déterminée par les points successifs d'échantillonnage des signaux *Vbip* et *Vuni,* a une allure de courbe, comme illustré Figure 8a (une courbe ouverte si l'échantillonnage se fait sur une fraction seulement du cycle cardiaque). En revanche, en cas de perte de capture, la caractéristique VGM est simplement constituée d'un nuage de points centrés approximativement autour du point de coordonnées {0,0}, comme illustré Figure 8b.

L'idée de base de l'invention consiste à définir un domaine D dans le repère {*Vuni, Vbip*} où est tracée la caractéristique VGM, à superposer ce domaine à la caractéristique VGM, à évaluer si cette caractéristique VGM est ou non contenue dans le domaine, et à décider selon le cas l'absence ou la présence d'une capture.

Le domaine D peut être avantageusement, comme illustré Figures 8a et 8b, un domaine rectangulaire centré sur le point de coordonnées {0,0}, de côtés 4 V (pour *Vbip*) x 1,5 V (pour *Vuni*).

Le critère de décision sera par exemple le suivant :

- si tous les points de la caractéristique VGM sont à l'intérieur de ce rectangle, alors il n'y a pas capture ;
- si au moins un point de cette caractéristique se situe hors du rectangle, alors il y a capture.

Des critères différents peuvent être utilisés, par exemple imposant la présence de deux points au moins de la caractéristique VGM hors du rectangle pour décider la présence d'une capture, voire même deux points consécutifs hors du rectangle : en particulier, dans ce dernier cas, la présence du pic de stimulation à l'intérieur de la fenêtre temporelle d'analyse n'est pas gênante.

On dispose ainsi d'un moyen très simple et très efficace de discriminer entre stimulation capturante et non-capturante, sans mise en oeuvre de techniques complexes d'analyse morphologique de la caractéristique VGM, ni comparaison de cette caractéristique à des modèles de référence préalablement acquis et nécessitant une mise à jour régulière.

Le test de capture peut se faire cycle à cycle, ou aussi bien être réalisés à intervalles réguliers, par exemple toutes les six heures.

Le résultat du test de capture peut être utilisé notamment pour vérifier qu'une thérapie a bien été délivrée sur les différents sites stimulés, notamment dans le cas d'une thérapie CRT où il est indispensable que les deux ventricules soient stimulés conjointement et/ou pour adapter si besoin les intervalles de stimulation (délai atrioventriculaire DAV et/ou délai interventriculaire DW) en fonction des résultats.

Il peut également servir à déterminer le seuil de stimulation et ajuster si besoin l'amplitude de l'impulsion de stimulation : pour ce faire, le dispositif applique alors à la cavité des impulsions de stimulation à énergie décroissante, et contrôle à chaque fois la présence ou non d'une onde évoquée par la méthode précédemment décrite. Si à une énergie donnée la capture est avérée, le dispositif considère que la stimulation est une stimulation efficace. L'énergie appliquée pour la stimulation suivante est réduite, typiquement d'un pas d'amplitude fixe, par exemple de 0,25 V. Dès que la capture est perdue, alors le dispositif considère que la stimulation est inefficace, et donc que le seuil de stimulation est supérieur à la dernière valeur appliquée. Le seuil de stimulation ainsi déterminé peut être conservé dans les mémoires de l'appareil, être transmis à un centre de recueil de données, ou encore utilisé par l'implant pour ajuster l'amplitude de la stimulation.

Pour d'autres détails sur les algorithmes d'ajustement de l'amplitude de stimulation à partir de tests de capture successifs, on pourra se référer notamment au EP 1 080 744 A1 (ELA Medical), qui décrit diverses techniques de mesure du seuil, de contrôle de cohérence des mesures et d'ajustement de la largeur et de l'amplitude de l'impulsion de stimulation ; les algorithmes qui y sont décrits peuvent être mis en oeuvre avec un test de capture effectué à partir d'une analyse de la caractéristique VGM effectuée selon les enseignements de la présente invention.

## Revendications

1. Un dispositif médical actif, comprenant :

   - des moyens de délivrance d'impulsions électriques de stimulation appliquées à des électrodes aptes à être implantées dans au moins une cavité cardiaque d'un patient ;
   - des moyens de recueil de l'activité électrique du coeur, comprenant des moyens pour produire au moins deux composantes temporelles distinctes *(Vbip, Vuni)* à partir de deux signaux EGM distincts d'électrogramme endocavitaire recueillis concurremment ; et
   - des moyens de test de capture, pour détecter la survenue d'une onde de dépolarisation induite par la stimulation de la cavité sur au moins un cycle stimulé, ces moyens de test de capture comprenant :

      • des moyens pour déterminer une caractéristique 2D non-temporelle (VGM) représentative du cycle cardiaque à analyser, à partir des variations de l'une des composantes temporelles (*Vuni*) en fonction de l'autre (*Vbip*); et
      • des moyens discriminateurs, pour déterminer la présence ou l'absence d'une capture par analyse de la caractéristique 2D non-temporelle,
      **caractérisé en ce que** les moyens discriminateurs comprennent :

         des moyens d'analyse topologique de la distribution des points de ladite la caractéristique 2D non-temporelle (VGM) par rapport à un domaine (D) prédéterminé défini dans un repère correspondant à l'espace des deux composantes temporelles, pour :

         • déterminer, sur la base de la position desdits points à l'intérieur ou à l'extérieur dudit domaine (D), si la caractéristique 2D non-temporelle (VGM) est ou non incluse dans ledit domaine (D), et

• décider i) l'absence de capture dans le premier cas et ii) la présence d'une capture dans le second cas.

2. Le dispositif de la revendication 1, dans lequel le domaine est un domaine rectangulaire.

3. Le dispositif de la revendication 2, dans lequel le domaine est centré sur le point d'origine du repère correspondant à l'espace des deux composantes temporelles.

4. Le dispositif de la revendication 1, dans lequel la caractéristique 2D non-temporelle est une caractéristique 2D échantillonnée décrite par une série de points discrets successifs, et dans lequel les moyens d'analyse topologique sont aptes à analyser la position relative de chaque point par rapport au domaine.

5. Le dispositif de la revendication 1, dans lequel les moyens d'analyse topologique sont aptes à décider que la caractéristique 2D n'est pas incluse dans le domaine dès lors qu'au moins un point de la caractéristique 2D échantillonnée se situe hors du domaine.

6. Le dispositif de la revendication 1, dans lequel les moyens d'analyse topologique sont aptes à décider que la caractéristique 2D n'est pas incluse dans le domaine dès lors qu'au moins deux points de la caractéristique 2D échantillonnée se situent hors du domaine.

7. Le dispositif de la revendication 1, dans lequel les moyens d'analyse topologique sont aptes à décider que la caractéristique 2D n'est pas incluse dans le domaine dès lors qu'au moins deux points consécutifs de la caractéristique 2D échantillonnée se situent hors du domaine.

8. Le dispositif de la revendication 1, dans lequel la caractéristique 2D non-temporelle est déterminée pour une pluralité de cycles cardiaques successifs, et dans lequel les moyens d'analyse topologique sont aptes à décider l'absence de capture si au moins l'une des caractéristiques ainsi déterminées est incluse dans le domaine, et la présence d'une capture dans le cas contraire.

9. Le dispositif de la revendication 1, dans lequel les moyens pour déterminer la caractéristique 2D non-temporelle sont des moyens aptes à déterminer cette caractéristique à partir des variations des composantes temporelles sur une fraction du cycle cardiaque à analyser, dans une fenêtre temporelle d'analyse ouverte à l'instant de la stimulation ou décalée par rapport à cet instant, de manière à isoler le complexe QRS du battement cardiaque des pics de stimulation ventriculaire et/ou auriculaire qui le précèdent.

10. Le dispositif de la revendication 9, pour un test de capture consistant à détecter la survenue d'une onde de dépolarisation induite dans une cavité par la stimulation de cette même cavité, dans lequel le dispositif comprend en outre :

- des moyens pour raccourcir temporairement le délai atrioventriculaire pendant l'activation des moyens de test de capture, de sorte qu'une contraction ventriculaire spontanée survenant après une absence de capture se situe hors de la fenêtre temporelle d'analyse.

11. Le dispositif de la revendication 9, pour un test de capture consistant à détecter la survenue d'une onde de dépolarisation induite dans une cavité par la stimulation d'une autre cavité, le dispositif comprenant en outre :

- des moyens pour raccourcir temporairement le délai atrioventriculaire pendant l'activation des moyens de test de capture ; et
- des moyens pour conditionner l'activation des moyens de test de capture au respect du critère : AR (ou PR) ≥ DAV + VV + M, AR (ou PR) étant l'intervalle séparant la stimulation (ou la détection) auriculaire de la dépolarisation ventriculaire subséquente, DAV étant la durée du délai atrioventriculaire, VV étant le délai de conduction entre les deux cavités, c'est-à-dire la durée que met la dépolarisation issue de la stimulation d'une cavité à se propager et être détectable dans la cavité non stimulée, et M étant une constante correspondant à la différence minimale admissible entre DAV+VV et AR (ou PR), de sorte qu'une contraction ventriculaire spontanée survenant après une absence de capture se situe hors de la fenêtre temporelle d'analyse.

12. Le dispositif de la revendication 1, dans lequel les signaux EGM recueillis concurremment sur des voies respectives distinctes comprennent :

- un signal *near-field* de composante bipolaire (*Vbip*) recueilli entre une électrode proximale et une électrode distale d'une sonde apte à être placée dans une cavité cardiaque ; et
- un signal *far-field* de composante unipolaire (*Vuni*) recueilli entre le boitier du dispositif et l'électrode proximale ou distale de la sonde.

**Patentansprüche**

1. Aktive medizinische Vorrichtung, welche aufweist:

    - Mittel zur Abgabe elektrischer Stimulationsimpulse, die an Elektroden angelegt werden, welche geeignet sind, in wenigstens einen Herzhohlraum eines Patienten implantiert zu werden;
    - Mittel zur Aufnahme der elektrischen Aktivität des Herzens, welche Mittel zum Erzeugen wenigstens zweier verschiedener Zeitkomponenten (*Vbip, Vuni*) ausgehend von zwei verschiedenen EGM-Signalen eines endokavitären Elektrogramms, die gleichzeitig aufgenommen werden, beinhalten; und
    - Capture-Test-Mittel, um das Auftreten einer Depolarisationswelle zu erfassen, welche durch die Stimulation des Hohlraums in wenigstens einem Stimulationszyklus induziert wird, wobei diese Capture-Test-Mittel enthalten:

        • Mittel zum Bestimmen eines nicht-zeitlichen 2D-Merkmals (VGM), das für den zu analysierenden Herzzyklus repräsentativ ist, ausgehend von den Schwankungen einer der Zeitkomponenten (*Vuni*) in Abhängigkeit von der anderen (*Vbip*); und
        · Diskriminatormittel, um das Vorhandensein oder die Abwesenheit eines Capture durch Analyse des nicht-zeitlichen 2D-Merkmals zu bestimmen,

    **dadurch gekennzeichnet, dass** die Diskriminatormittel enthalten:
    Mittel zur topologischen Analyse der Verteilung der Punkte des nicht-zeitlichen 2D-Merkmals (VGM) in Bezug auf einen vorbestimmten Bereich (D), der in einem Koordinatensystem definiert ist, das dem Raum der zwei Zeitkomponenten entspricht, um:

        · zu bestimmen, auf der Basis der Position der Punkte innerhalb oder außerhalb des Bereichs (D), ob das nicht-zeitliche 2D-Merkmal (VGM) in dem Bereich (D) enthalten ist oder nicht, und
        · zu entscheiden, dass i) kein Capture vorhanden ist, im ersten Fall, und ii) ein Capture vorhanden ist, im zweiten Fall.

2. Vorrichtung nach Anspruch 1, wobei der Bereich ein rechteckiger Bereich ist.

3. Vorrichtung nach Anspruch 2, wobei der Bereich um den Ursprungspunkt des Koordinatensystems zentriert ist, das dem Raum der zwei Zeitkomponenten entspricht.

4. Vorrichtung nach Anspruch 1, wobei das nicht-zeitliche 2D-Merkmal ein abgetastetes 2D-Merkmal ist, das durch eine Reihe von aufeinander folgenden diskreten Punkten beschrieben wird, und wobei die Mittel zur topologischen Analyse geeignet sind, die relative Position jedes Punktes bezüglich des Bereichs zu analysieren.

5. Vorrichtung nach Anspruch 1, wobei die Mittel zur topologischen Analyse geeignet sind zu entscheiden, dass das 2D-Merkmal nicht in dem Bereich enthalten ist, sofern sich wenigstens ein Punkt des abgetasteten 2D-Merkmals außerhalb des Bereichs befindet.

6. Vorrichtung nach Anspruch 1, wobei die Mittel zur topologischen Analyse geeignet sind zu entscheiden, dass das 2D-Merkmal nicht in dem Bereich enthalten ist, sofern sich wenigstens zwei Punkte des abgetasteten 2D-Merkmals außerhalb des Bereichs befinden.

7. Vorrichtung nach Anspruch 1, wobei die Mittel zur topologischen Analyse geeignet sind zu entscheiden, dass das 2D-Merkmal nicht in dem Bereich enthalten ist, sofern sich wenigstens zwei aufeinander folgende Punkte des abgetasteten 2D-Merkmals außerhalb des Bereichs befinden.

8. Vorrichtung nach Anspruch 1, wobei das nicht-zeitliche 2D-Merkmal für mehrere aufeinander folgende Herzzyklen bestimmt wird, und wobei die Mittel zur topologischen Analyse geeignet sind zu entscheiden, dass kein Capture vorhanden ist, falls wenigstens eines der so bestimmten Merkmale in dem Bereich enthalten ist, und dass ein Capture vorhanden ist, im entgegengesetzten Fall.

9. Vorrichtung nach Anspruch 1, wobei die Mittel zum Bestimmen des nicht-zeitlichen 2D-Merkmals Mittel sind, die geeignet sind, dieses Merkmal ausgehend von den Schwankungen der Zeitkomponenten über einen Bruchteil des zu analysierenden Herzzyklus in einem Analysezeitfenster zu bestimmen, das zum Zeitpunkt der Stimulation offen ist oder bezüglich dieses Zeitpunktes versetzt ist, um den QRS-Komplex des Herzschlags von den Peaks der ventrikulären und/oder aurikulären Stimulation zu isolieren, die ihm vorausgehen.

10. Vorrichtung nach Anspruch 9 für einen Capture-Test, der darin besteht, das Auftreten einer Depolarisationswelle zu erfassen, die in einem Hohlraum durch die Stimulation dieses Hohlraums selbst hervorgerufen wird, wobei die Vorrichtung außerdem aufweist:

    - Mittel zum Verkürzen der atrioventrikulären Überleitungszeit während der Aktivierung der Capture-Test-Mittel,

derart, dass sich eine spontane ventrikuläre Kontraktion, die nach einer Abwesenheit eines Capture auftritt, außerhalb des Analysezeitfensters befindet.

11. Vorrichtung nach Anspruch 9 für einen Capture-Test, der darin besteht, das Auftreten einer Depolarisationswelle zu erfassen, die in einem Hohlraum durch die Stimulation eines anderen Hohlraums hervorgerufen wird, wobei die Vorrichtung außerdem aufweist:

- Mittel zum Verkürzen der atrioventrikulären Überleitungszeit während der Aktivierung der Capture-Test-Mittel; und
- Mittel, um die Aktivierung der Capture-Test-Mittel von der Erfüllung des folgenden Kriteriums abhängig zu machen: AR (oder PR) ≥ DAV + VV + M,

wobei AR (oder PR) das Intervall ist, welches die aurikuläre Stimulation (oder die aurikuläre Erfassung) von der nachfolgenden ventrikulären Depolarisation trennt,

wobei DAV die Dauer der atrioventrikulären Überleitungszeit ist,

wobei VV die Leitungsverzögerung zwischen den zwei Hohlräumen ist, das heißt die Dauer, welche die durch die Stimulation eines Hohlraums verursachte Depolarisation benötigt, um sich auszubreiten und in dem nicht stimulierten Hohlraum erfassbar zu sein, und

wobei M eine Konstante ist, die der minimalen zulässigen Differenz zwischen DAV+VV und AR (oder PR) entspricht,

derart, dass sich eine spontane ventrikuläre Kontraktion, die nach einer Abwesenheit eines Capture auftritt, außerhalb des Analysezeitfensters befindet.

12. Vorrichtung nach Anspruch 1, wobei die EGM-Signale, die gleichzeitig auf unterschiedlichen jeweiligen Kanälen aufgenommen werden, enthalten:

- ein Nahfeldsignal einer bipolaren Komponente (*Vbip*), das zwischen einer proximalen Elektrode und einer distalen Elektrode einer Sonde aufgenommen wird, die geeignet ist, in einem Herzhohlraum angebracht zu werden; und
- ein Fernfeldsignal einer unipolaren Komponente (*Vuni*), das zwischen dem Gehäuse der Vorrichtung und der proximalen oder distalen Elektrode der Sonde aufgenommen wird.

**Claims**

1. An active medical device, comprising:

- means for delivering electric stimulation pulses applied to electrodes adapted to be implanted in at least one heart cavity of a patient;
- means for collecting the electric activity of the heart, comprising means for producing at least two distinct time components (*Vbip*, *Vuni*) based on two distinct endocavitary electrogram EGM signals collected concurrently; and
- capture test means, for detecting the occurrence of a depolarization wave induced by the stimulation of the cavity over at least one stimulated cycle, such capture test means comprising:

· means for determining a non-time 2D characteristic (VGM) representative of the cardiac cycle to be analysed, based on the variations of one of the time components (*Vuni*) as a function of the other (*Vbip*); and
· discriminating means, for determining the presence or the absence of a capture by analysing the non-time 2D characteristic,

**characterized in that** the discriminating means comprise:

means for the topological analysis of the distribution of the points of said non-time 2D characteristic (VGM) with respect to a predetermined domain (D) defined in a reference system corresponding to the two time components space, for :

· determining, based on the position of said points inside or outside said domain (D), whether the non-time 2D characteristic (VGM) is included or not in said domain (D), and
· deciding i) the absence of capture in the first case and ii) the presence of a capture in the second case.

2. The device of claim 1, wherein the domain is a rectangular domain.

3. The device of claim 2, wherein the domain is centred to the point of origin of the reference system corresponding to the two time components space.

4. The device of claim 1, wherein the non-time 2D characteristic is a sampled 2D characteristics described by a series of successive discrete points, and wherein the topological analysis means are adapted to analyse the relative position of each point with respect to the domain.

5. The device of claim 1, wherein the topological analysis means are adapted to decide that the 2D characteristic is not included in the domain as soon as at least one point of the sampled 2D characteristic is located outside the domain.

6. The device of claim 1, wherein the topological analysis means are adapted to decide that the 2D characteristic is not included in the domain as soon as at least two points of the sampled 2D characteristic are located outside the domain.

7. The device of claim 1, wherein the topological analysis means are adapted to decide that the 2D characteristic is not included in the domain as soon as at least two consecutive points of the sampled 2D characteristic are located outside the domain.

8. The device of claim 1, wherein the non-time 2D characteristic is determined for a plurality of successive cardiac cycles, and wherein the topological analysis means are adapted to decide the absence of capture if at least one of the so-determined characteristics is included in the domain, and the presence of a capture in the opposite case.

9. The device of claim 1, wherein the means for determining the non-time 2D characteristic are means adapted to determine this characteristic based on the variations of the time components over a fraction of the cardiac cycle to be analysed, in a time window of analysis open at the time instant of the stimulation or offset with respect to this time instant, so as to isolate the QRS complex of the heart beat from the ventricular and/or atrial stimulation peaks that precede it.

10. The device of claim 9, for a capture test consisting in detecting the occurrence of a depolarization wave induced in a cavity by the stimulation of this same cavity, wherein the device further comprises:

    - means for temporarily reducing the atrioventricular delay during the activation of the capture test means,
    so that a spontaneous ventricular contraction occurring after an absence of capture is located outside the time window of analysis.

11. The device of claim 9, for a capture test consisting in detecting the occurrence of a depolarization wave induced in a cavity by the stimulation of another cavity, the device further comprising:

    - means for temporarily reducing the atrioventricular delay during the activation of the capture test means; and
    - means for conditioning the activation of the capture test means to the fulfilling of the criterion: AR (or PR) $\geq$ DAV + VV + M,
    wherein:

    AR (or PR) is the interval separating the atrial stimulation (or the detection) from the subsequent ventricular depolarization,
    DAV is the duration of the atrioventricular delay,
    VV is the delay of conduction between the two cavities, i.e. the duration taken by the depolarization coming from the stimulation of a cavity to propagate and to be detectable in the non-stimulated cavity, and
    M is a constant corresponding to the minimum allowable difference between DAV + VV and AR (or PR),

    so that a spontaneous ventricular contraction occurring after an absence of capture is located outside the time window of analysis.

12. The device of claim 1, wherein the EGM signals collected concurrently on respective distinct paths comprise:

    - a near-field signal of bipolar component *(Vbip)* collected between a proximal electrode and a distal electrode of a lead adapted to be placed in a cardiac cavity ; and
    - a far-field signal of unipolar component *(Vuni)* collected between the casing of the device and the proximal or distal electrode of the lead.

Fig. 1

(a)

(b)

Fig. 2

Fig. 3

Fig. 4

Fig. 5

Fig. 6

Fig. 7

Fig. 8a

Fig. 8b

**EP 2 742 971 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 9302741 A1
- US 5411533 A
- EP 1287849 A1
- EP 2324885 A1
- EP 1080744 A1